**Europäisches Patentamt**

**European Patent Office**

**Office européen des brevets**

(19)

(11) Publication number: **0 007 932**

**B1**

(12) # EUROPEAN PATENT SPECIFICATION

(45) Date of publication of patent specification: **24.08.83**

(21) Application number: **78900004.9**

(22) Date of filing: **01.06.78**

(86) International application number:
**PCT/SE78/00001**

(87) International publication number:
**WO 78/00005 07.12.78 Gazette 78/8**

(51) Int. Cl.³: **A 61 K 9/00, A 61 K 9/14, A 61 K 47/00, A 61 K 33/26**

(54) **MAGNETIC POLYMER PARTICLES.**

(30) Priority: **02.06.77 SE 7706431**

(43) Date of publication of application:
**20.02.80 Bulletin 80/4**

(45) Publication of the grant of the patent:
**24.08.83 Bulletin 83/34**

(84) Designated Contracting States:
**FR**

(56) References cited:
**DE - A - 1 467 974**
**DE - A - 2 150 581**
**DE - A - 2 217 266**
**DE - A - 2 654 723**
**FR - M - 7 403**
**US - A - 4 018 886**

**Methods in Enzymology, Volume XLIV, 1976,**
**"Immobilized Enzymes", p. 216, 278, 324—326**

(73) Proprietor: **MOSBACH, Klaus H.**
**Lackalänga 31-38**
**S-240 20 Furulund (SE)**

(72) Inventor: **MOSBACH, Klaus H.**
**Lackalänga 31-38**
**S-240 20 Furulund (SE)**

(74) Representative: **Wallin, John Erik et al,**
**AWAPATENT AB Box 5117**
**S-200 71 Malmö (SE)**

(56) References cited:
**Biotechnology and Bioengineering Sym. 3**
**(1972) pages 173—174**

**Biotechnology and Bioengineering XV, (1973),**
**603—606; XVI, (1974) 987—990**

Courier Press, Leamington Spa, England

## Magnetic polymer particles

This invention relates to magnetic polymer particles and the process for their production and utilization.

More particularly, the invention relates to magnetic polymer particles as carriers of biologically, preferably pharmaceutically, active substances, the utilization of the magnetic polymer particles, and the method of retaining a biologically active substance, preferably a pharmaceutically active substance within a restricted area.

It is known to use precipitated iron oxide mixed with polyacrylic amide, which is then further polymerized, as carrier for biologically active substances, see Biotechnol. & Bioeng., XVI, (1974), 987—990. However, the magnetic amide gel thus produced has a poorer capacity that generally used non-magnetic, porous agarose, when used in affinity chromatography. Further Biotechnol. & Bioeng. Sym., 3 (1972), 173—174, teaches an anticoagulant containing microcapsules which may include various substances, such as magnetite, enzymes, indicators and necessary cofactors. In Biotechnol. & Bioeng., XV, (1973), 603—606, there is described immobilization of enzymes by direct attachment to magnetite particles There is also described precipitated iron oxide mixed with cellulose for the formation of iron oxide-cellulose particles to which enzyme materials are coupled.

Magnetic polymer particles for use as carriers of biologically active substances, preferably pharmaceutically active substances, should be useful within many technical and medicinal fields because they can be concentrated by the application of a magnetic field.

This invention provides magnetic polymer particles for use as carriers of biologically, preferably pharmaceutically active substance, said particles being particles of a porous polymer in the pores or lattice of which colloidal ferrite is deposited, said biologically active substance being associated with the polymer particles.

Further, the invention comprises a process for production of magnetic polymer particles for use as carriers of biologically, preferably pharmaceutically active substance, in which method porous polymer particles which may carry the biologically active substance, and/or polymerizable monomers are contacted with a colloidal solution of ferrite and, optionally, biologically active substance, the optionally present monomers as polymerized, the solvent is optionally removed, the colloidal ferrite and the optionally present biologically active substance being deposited in the pores or lattice of the polymer particles, whereupon the resulting magnetic polymer particles are contacted with the biologically active substance, if the said substance is not already associated with the polymer particles.

The invention also comprises the utilization of the magnetic polymer particles as carriers of biologically active substance.

The invention in addition provides a method of retaining a biologically active substance within a restricted area, in which method the substance is associated with a carrier in the form of porous polymer particles to which magnetic properties are imparted by incorporation of colloidal ferrite, and the magnetic carrier with the substance associated therewith is then retained within the restricted area by application of a magnetic field.

As polymer use can be made for instance of a polysaccharide, preferably agarose or starch. Another suitable polymer is acrylic polymer. The polymer should be biocompatible. A particular advantage, when used in the treatment of mammals, is gained if the polymer is biodegradable, such as starch which is enzymatically degraded in the organism. Polymers forming too small lattices are unsuitable.

The magnetic material is used in the form of a colloidal solution of ferrite. Colloidal ferrite is commercially available as ultramicroscopical (about 100 Å) ferrite particles surrounded by a thin layer (about 25 Å) of a polymer. The particles are prevented by said polymer layer from adhering to each other in a magnetic field. Random collisions (Brownian motion) with the molecules of the carrier liquid retain the particles in colloidal solution.

According to this invention the magnetic polymer particles which contain colloidal ferrite are produced in a very advantageous manner by contacting already formed porous polymer which may have associated biologically active substance, with a colloidal solution of ferrite. After optional removal of the solvent of the colloidal ferrite, said colloidal ferrite remains in the polymer, probably by precipitation in the pores or lattice of the polymer, whereby the polymer obtains magnetic properties. In this manner, polymer particles already produced can be made magnetical afterwards. Polymers which are already substituted with biologically active substances are commercially available, which makes this method extremely advantageous. If the polymer which has thus been made magnetic does not already have associated biologically active substances, said polymer is then contacted with a biologically active substance which is thereby associated with the polymer serving as a carrier of the substance.

In another embodiment of the production process according to the invention, magnetic polymer particles are produced by polymerization of say acrylic monomers in presence of a colloidal solution of ferrite, said colloidal ferrite remaining in the resulting polymeric lattice. Another way is to contact say acrylic monomers simultaneously with the colloidal solution of ferrite and agarose, the acrylic monomers being polymerized. To the magnetic polymer particles thus formed, biologically active substances can be associated for instance by the so-called BrCN-method.

In a further embodiment of the production process according to the invention the polymer particles are produced by polymerization of a monomer in the simultaneous presence of a cross-linking agent, the colloidal solution of ferrite and the biologically active substance. In this process both the colloidal ferrite and the biologically active substance are locked up in the polymeric lattice. This process can be performed for instance by polymerization of alkyl monomers in the presence of cross-linking agent, enzyme (such as trypsin) and Ferrofluid (colloidal solution of ferrite particles, trademark).

The magnetic polymeric particles according to the invention, which contain colloidal ferrite, can be used for instance at so-called affinity chromatography, for instance to separate enzymes from a mixture of several different enzymes. To this end, for instance an enzyme inhibitor which may be either a specific inhibitor or a general inhibitor or cofactor, is associated with the magnetic polymer particles. By specific inhibitor is meant an inhibitor specific to but one enzyme, but this is the case with most inhibitors, whereas a general inhibitor can inhibit several different enzymes. When these polymer particles with inhibitors associated therewith are added to a solution of several different enzymes, the inhibitors will bind the enzymes, and after agitation for some time it is possible to separate the contemplated enzyme or enzymes by applying a magnetic field within or without the solution. The magnetic particles will collect at the magnet and the remainder of the solution can be removed. The enzymes can then be separated from the inhibitors bound to the magnetic carrier by methods well-known to those skilled in the art. As an example of this use there may be mentioned postmagnetized Sepharose (Pharmacia, Sweden) to which the general inhibitor adenosine monophosphate (AMP) has been adsorbed. Moreover, other enzyme systems, such as glucose-6-phosphate dehydrogenase which has been adsorbed to postmagnetized 2',5'-ADP-Sepharose (Pharmacia, Sweden), have been successfully employed.

The magnetic polymer particles according to the invention can further be used in immuno adsorbance. Antibodies against human serum albumin are thereby associated with Sepharose (Pharmacia, Sweden) and are postmagnetized by treatment with a colloidal solution of ferrite. With the aid of the antibodies thus supported on a magnetic carrier human serum albumin can be collected.

As mentioned in the foregoing, particular advantages are gained with the use of a colloidal solution, int. al. because — as has also been mentioned in the foregoing — polymers already formed can be postmagnetized.

The present invention also provides a very advantageous method of retaining a biologically, preferably pharmaceutically active substance within a restricted area, it being necessary to utilize a much smaller amount of the active substance than would have been the case with the same substance without application of the method of the invention. Further there is no risk of the magnetic polymer particles clogging together when used in treatment of deceased tissues.

As an example of this use there may be mentioned the treatment of thrombi. Proteolytic enzymes, such as plasmine, are known by their ability to dissolve different substrates, among them fibrin (thrombi). By associating these proteolytic enzymes with or enclosing them in the magnetic polymer particles according to the present invention the enzymes can be enriched and retained in position *in vivo* with the aid of an outer or an inner magnetic field. This will provide a local concentration of the active substance in the treatment of thrombi, whereby the dosis of the proteolytic enzyme can be considerably reduced in relation to the dosis utilized in hitherto known methods.

In a manner analogous to that applied in the treatment of thrombi a general local medicinal treatment can be realized, the active substance, for instance a cytostaticum, being either coupled via a reversible weak bond to the carrier and being then released on the spot where it shall become active, or be fixedly coupled to the carrier, being active in a bound state. In this way a high concentration of the active substance is obtained at the desired spot simultaneously as injurious effects of the active substance in other parts of the organism are avoided.

A further medicinal use is the so-called slow-release of deposits of active substance. Great advantages can be gained by supply of for instance insulin or steroids bound to or enclosed in magnetic polymer particles according to the invention, as deposits, the active substances being retained in the organism by the application of a magnetic field and being permitted slowly to diffuse out.

A further field in which the magnetic particles according to the present invention can be employed is in general chemical, particularly organo-chemical reactions in which the substances to be reacted are first associated with a magnetic carrier according to the present invention, whereupon a very intimate contact is realized between the substances associated with the carrier by the application of a magnetic field. This will produce a very high specific reaction without any secondary reactions, that is, very high efficiency, and it is possible to work at low concentrations. Further, an interaction between two or more components can be forced by the application of a magnetic field.

In another very promising embodiment of the present invention live microbes have been successfully baked into gels of polymeric materials such as polysaccharides, together with a colloidal ferrite solution (Ferrofluid). Magnetic immobilized microbes are thereby obtained.

By treating red corpuscles with a colloidal ferrite solution there are obtained highly biocompatible carriers of biological substance. The desired substances can then be associated with these carriers. The initially kidney-shaped corpuscles will become round. Liposomes can also be treated with the colloidal ferrite solution, said solution penetrating into the onion skin-shaped layers of fatty acids and water in the liposomes.

A process for production of magnetic polymer particles according to the invention is illustrated by the following example which should not be considered restrictive to the invention. As will be realized by one of ordinary skill in the art, numerous changes and modifications can be realized without departing from the inventive idea, such as it is defined in the present description and the appended claims.

### Example

One gram of moist 5'-AMP- (or 2',5'-ADP)-Sepharose-4B (Pharmacia) which was previously swollen in 0,1 M sodium phosphate buffer pH 7.5, and washed with $H_2O$ was packed in a column. Subsequently 6.5 ml of ferrofluid (base, $H_2O$; magnetic saturation, 200 G; trademark AO5, from Ferrofluidics Corporation, Burlington, Mass., USA) were pumped through the column and cycled for 4 h at a flow rate of 50 ml $h^{1-}$ (usually at room temperature). After washing with 1 l of $H_2O$, on glass filter, incubation overnight at 4°C with 100 mg of bovine serum albumin in 0,1 M Tris-HCl buffer pH 7.6, 5 mM in EDTA and 1 mM 2-mercaptoethanol was carried out, followed by washing with 200 ml 1 M NaCl and 200 ml of the same buffer. The dark brown gel beads were then magnetic and ready for use.

Whether the ferrite particles simply adsorb to the gel or precipitate out in the interior of the beads remains to be established. (In this context it should be noted that entrapment of ferrofluid within the lattice of polyacrylamide can easily be accomplished.) In any case, the magnetic properties of the gel remain unchanged after extensive washing with buffer (even washing with, for example, ethanol or 40% ethylene glycol did not remove the ferrite particles) and repeated use in magnetic fields; they are strong enough to allow rapid sedimentation even when applying weak permanent magnets.

### Claims

1. Magnetic polymer particles for use as carriers of biologically active substance, characterised in that they comprise particles of a porous polymer in the pores or lattice of which there is deposited colloidal ferrite, said biologically active substance being associated with the polymer particles.

2. Magnetic polymer particles as claimed in claim 1, characterised in that the biologically active substance is a pharmaceutically active substance.

3. Magnetic polymer particles as claimed in claim 1 or 2, characterised in that the biologically active substance is an enzyme inhibitor, an enzyme, a cystostaticum, a radio nucleotid, insulin, a steroid or a live microbe.

4. Magnetic polymer particles as claimed in claim 3, characterised in that, when the biologically active substance is an enzyme, it is a proteolytic enzyme.

5. Magnetic polymer particles as claimed in any of claims 1—4, characterised in that the polymer is chosen from the group consisting of an acrylic polymer, red corpuscles, a liposome and a polysaccharide.

6. Magnetic polymer particles as claimed in claim 5, characterised in that, when the polymer is a polysaccharide, it is agarose or starch.

7. A process for production of magnetic polymer particles for use as carriers of biologically active substance, characterised in that the porous polymer particles which may carry the biologically active substance, and/or polymerizable monomers are contacted with a colloidal solution of ferrite and optionally biologically active substance, the optionally present monomers are polymerized, the solvent is optionally removed, the colloidal ferrite and the optionally present biologically active substance being deposited in the pores or lattice of the polymer particles, whereupon the resulting magnetic polymer particles are contacted with the biologically active substance, if the said substance is not already associated with the polymer particles.

8. A process as claimed in claim 7, characterised in that the biologically active substance is a pharmaceutically active substance.

9. A process as claimed in claim 7 or 8, characterised in that there is associated with the magnetic polymer particles as biologically active substance an enzyme inhibitor, an enzyme, a cytostaticum, a nucleotid, insulin, a steroid or a live microbe.

10. A process as claimed in claim 9, characterised in that, when the biologically active substance is an enzyme, it is a proteolytic enzyme.

11. A process as claimed in any of claims 7—10, characterised in that as polymer particles use is made of particles chosen from the group consisting of red corpuscles, particles of a liposome, an acrylic polymer and a polysaccharide.

12. A process as claimed in claim 11, characterised in that, when the polymer is a polysaccharide, it is agarose or starch.

13. A process as claimed in claim 7, charac-

## 0 007 932

terised in that an acrylic monomer is inserted as polymerizable monomer.

14. Use of the magnetic polymer particles claimed in claim 1, as carriers of biologically active substance.

15. A method of retaining a biologically active substance within a restricted area in chemical reactions, affinity chromatography or immunoadsorbance, characterised in that the substance is associated with a carrier in the form of porous polymer particles which are given magnetic properties by incorporation of colloidal ferrite, whereupon the magnetic polymer particles with the substance associated therewith is retained within the restricted area by the application of a magnetic field.

## Revendications

1. Particules magnétiques de polymère pour l'emploi comme supports d'une substance à activité biologique, caractérisées en ce qu'elles comprennent des particules d'un polymère poreux dans les pores ou le réseau duquel un ferrite colloïdal est déposé, ladite substance à activité biologique étant associée aux particules de polymère.

2. Particules magnétiques de polymère comme revendiqué dans la revendication 1, caractérisées en ce que la substance à activité biologique est une substance à activité pharmaceutique.

3. Particules magnétiques de polymère comme revendiqué dans la revendication 1 ou 2, caractérisées en ce que la substance à activité biologique est un inhibiteur enzymatique, une enzyme, un cytostatique, un radionucléotide, l'insuline, un stéroïde ou un microbe vivant.

4. Particules magnétiques de polymère comme revendiqué dans la revendication 3, caractérisées en ce que, lorsque la substance à activité biologique est une enzyme, c'est une enzyme protéolytique.

5. Particules magnétiques de polymère comme revendiqué dans l'une quelconque des revendications 1 à 4, caractérisées en ce que le polymère est choisi parmi le groupe constitué par un polymère acrylique, des globules rouges, un liposome et un polysaccharide.

6. Particules magnétiques de polymère comme revendiqué dans la revendication 5, caractérisées en ce que, lorsque le polymère est un polysaccharide, c'est l'agarose ou l'amidon.

7. Un procédé pour la production de particules magnétiques de polymère pour l'emploi comme supports d'une substance à activité biologique, caractérisé en ce que les particules de polymère poreux qui peuvent porter la substance à activité biologique, et/ou des monomères polymérisables, sont mis en contact avec une solution colloïdale de ferrite et éventuellement une substance à activité biologique, les monomères éventuellement présents sont polymérisés, le solvant est éventuellement éliminé, le ferrite colloïdal et la substance à activité biologique éventuellement présente étant déposés dans les pores ou dans le réseau des particules de polymère, après quoi les particules magnétiques de polymère obtenues sont mises en contact avec la substance à activité biologique, si ladite substance n'a pas déjà été associée aux particules de polymère.

8. Un procédé comme revendiqué dans la revendication 7, caractérisé en ce que la substance à activité biologique est une substance à activité pharmaceutique.

9. Un procédé comme revendiqué dans la revendication 7 ou 8, caractérisé en ce que sont associés aux particules magnétiques de polymère, comme substance à activité biologique, un inhibiteur enzymatique, une enzyme, un cytostatique, un nucléotide, l'insuline, un stéroïde ou un microbe vivant.

10. Un procédé comme revendiqué dans la revendication 9 caractérisé en ce que, lorsque la substance à activité biologique est une enzyme, c'est une enzyme protéolytique.

11. Un procédé comme revendiqué dans l'une quelconque des revendications 7 à 10, caractérisé en ce que, comme particules de polymère, on utilise des particules choisies parmi le groupe constitué par les globules rouges, les particules d'un liposome, un polymère acrylique et un polysaccharide.

12. Un procédé comme revendiqué dans la revendication 11, caractérisé en ce que, lorsque le polymère est un polysaccharide, c'est l'agarose ou l'amidon.

13. Un procédé comme revendique dans la revendication 7 caractérisé en ce qu'un monomère acrylique est inséré comme monomère polymérisable.

14. Emploi des particules magnétiques de polymères revendiquées dans la revendication 1 comme supports d'une substance à activité biologique.

15. Un procédé de rétention d'une substance à activité biologique dans une zone limitée dans des réactions chimiques, en chromatographie d'affinité ou en immunoadsorption, caractérisé en ce que la substance est associée à un support sous forme de particules de polymère poreux auxquelles sont conférées des propriétés magnétiques par incorporation de ferrite colloïdal après quoi les particules magnétiques de polymère avec la substance qui leur est associée sont retenues dans la zone limitée par l'application d'un champ magnétique.

## Patentansprüche

1. Magnetische Polymerpartikel zur Verwendung als Träger biologisch wirksamer Substanz, dadurch gekennzeichnet, dass sie Partikel eines porösen Polymers umfassen, in dessen Poren oder Gitter kolloidales Ferrit abgelagert ist, wobei die biologisch wirksame Substanz mit den Polymerpartikeln assoziiert ist.

2. Magnetische Polymerpartikel nach Anspruch 1, dadurch gekennzeichnet, dass die biologisch wirksame Substanz eine pharmazeutisch wirksame Substanz ist.

3. Magnetische Polymerpartikel nach Anspruch 1 oder 2, dadurch gekennzeichnet, dass die biologisch wirksame Substanz ein Enzyminhibitor, ein Enzym, ein Cytostaticum, ein Radionucleotid, Insulin, ein Steroid oder eine lebende Mikrobe ist.

4. Magnetische Polymerpartikel nach Anspruch 3, dadurch gekennzeichnet, dass die biologisch wirksame Substanz, wenn sie ein Enzym ist, ein proteolytisches Enzym ist.

5. Magnetische Polymerpartikel nach einem der Ansprüche 1—4, dadurch gekennzeichnet, dass das Polymer aus der aus einem Acrylpolymer, roten Blutkörperchen, einem Liposom und einem Polysaccharid bestehenden Gruppe gewählt ist.

6. Magnetische Polymerpartikel nach Anspruch 5, dadurch gekennzeichnet, dass das Polymer, wenn es ein Polysaccharid ist, Agarose oder Stärke ist.

7. Verfahren zur Herstellung magnetischer Polymerpartikel zur Verwendung als Träger biologisch wirksamer Substanz, dadurch gekennzeichnet, dass die porösen Polymerpartikel, welche die biologisch wirksame Substanz tragen können, und/oder polymerisierbare Monomere mit einer kolloidalen Lösung aus Ferrit und der ggf. anwesenden biologisch wirksamen Substanz in Berührung gebracht werden, dass die ggf. anwesenden Monomere polymerisiert werden, und dass das Lösungsmittel ggf. beseitigt wird, wobei das kolloidale Ferrit und die ggf. anwesende, biologische Wirksame Substanz in den Poren oder dem Gitter der Polymerpartikel abgelagert werden, wonach die gebildeten magnetischen Polymerpartikel mit der biologisch wirksamen Substanz in Berührung gebracht werden, falls diese Substanz nicht bereits mit den Polymerpartikeln assoziiert ist.

8. Verfahren nach Anspruch 7, dadurch gekennzeichnet, dass die biologisch wirksame Substanz eine pharmazeutisch wirksame Substanz ist.

9. Verfahren nach Anspruch 7 oder 8, dadurch gekennzeichnet, dass mit den magnetischen Polymerpartikeln als biologisch wirksame Substanz eine Enzyminhibitor, ein Enzym, ein Cytostaticum, ein Nucleotid, Insulin, Ein Steroid oder eine lebende Mikrobe assoziiert wird.

10. Verfahren nach Anspruch 9, dadurch gekennzeichnet, dass die biologisch wirksame Substanz, falls sie ein Enzym ist, ein proteolytisches Enzym ist.

11. Verfahren nach einem der Ansprüche 7—10, dadurch gekennzeichnet, dass als Polymerpartikel Partikel benutzt werden, die aus einem Acrylpolymer, roten Blutkörperchen, einem Liposom und einem Polysaccharid bestehen.

12. Verfahren nach Anspruch 11, dadurch gekennzeichnet, dass das Polymer, falls es ein Polysaccharid ist, Agarose oder Stärke ist.

13. Verfahren nach Anspruch 7, dadurch gekennzeichnet, dass also polymerisierbares Monomer ein Acrylmonomer eingesetzt wird.

14. Anwendung der magnetischen Polymerpartikel nach Anspruch 1 als Träger biologisch wirksamer Substanz.

15. Verfahren zum Festhalten einer biologische wirksamen Substanz innerhalb eines begrenzten Bereichs in chemischen Reaktionen, Affinitätschromatographie oder in der Immunadsorptionstechnik, dadurch gekennzeichnet, dass die Substanz mit einem Träger in der Form von porösen Polymerpartikeln assoziiert wird, denen durch Einverleibung kolloidalen Ferrits magnetische Eigenschaften verliehen worden sind, wonach die magnetischen Polymerpartikel und die damit assoziierte Substanz durch Anbringung eines magnetischen Felds innerhalb des begrenzten Bereichs festgehalten werden.